# FASCICULE DE BREVET EUROPEEN

(11) **EP 0 687 419 B1**
(45) Date de publication et mention de la délivrance du brevet: **23.01.2002**
(21) Numéro de dépôt: 94109356.9
(22) Date de dépôt: 17.06.1994
(51) Int. Cl.: A23D 9/06, C11B 5/00, A23J 7/00, A61K 7/00

(54) **Incorporation dans un lipide d'un principe actif hydrosoluble**
Aufnahme eines wasserlöslichen Wirkstoffes in einem Lipid
Incorporation of a watersoluble compound into a lipid

(43) Date de publication de la demande: 20.12.1995
(73) Titulaire: SOCIETE DES PRODUITS NESTLE S.A., 1800 Vevey (CH)
(72) Inventeur: Bertholet, Raymond, CH-1807 Blonay (CH)
(74) Mandataire: Archambault, Jean

(56) Documents cités:
- EP-A- 0 365 868
- EP-A- 0 422 543
- WO-A-90/15537
- FR-A- 2 627 385
- US-A- 3 701 668
- US-A- 5 084 289
- DATABASE WPI Week 8813, Derwent Publications Ltd., London, GB; AN 88-087108 & JP-A-63 036 792 (NIPPON OILS & FATS KK) 17 Février 1988 & PATENT ABSTRACTS OF JAPAN vol. 012, no. 248 (C-511) 13 Juillet 1988 & JP-A-63 036 792 (NIPPON OILS & FATS CO LTD) 17 Février 1988
- DATABASE WPI Week 8716, Derwent Publications Ltd., London, GB; AN 87-112992 & JP-A-62 059 287 (QP CORP) 14 Mars 1987

## Description

L'invention concerne un procédé d'incorporation dans un lipide d'un principe actif hydrosoluble, notamment de mise sous forme stable d'un antioxydant hydrosoluble dans une phase lipidique.

La plupart des huiles et certaines graisses utilisées dans les produits alimentaires, cosmétiques et pharmaceutiques sont riches en acides gras polyinsaturés et sont, de ce fait, particulièrement sensibles à l'oxydation. Leur stabilité peut être améliorée par l'adjonction d'antioxydants synthétiques tels que, par exemple, le BHA (butyl hydroxyanisol), le BHT (butyl hydroxytoluène) ou le TBHQ (tertiobutyl hydroquinone). Cependant, l'inocuité de ces composés est mise en doute.

On cherche à les remplacer par des composés antioxydants naturels, soit liposolubles comme, par exemple, les tocophérols ou le palmitate d'ascorbyle, soit hydrosolubles comme, par exemple, l'acide ascorbique, les extraits végétaux, les acides organiques ou les acides aminés. Dans les cas des composés hydrosolubles, l'utilisation d'un agent émulsifiant comme, par exemple, un phospholipide est nécessaire pour incorporer les antioxydants hydrosolubles dans les huiles sous forme de micelles.

De par leur structure, les phospholipides sont capables de créer des associations avec certains composés hydrosolubles pour former des micelles, qui elles sont liposolubles.

L'incorporation de vitamine C ou d'autres composés hydrosolubles dans les huiles par l'intermédiaire de phospholipides est connue, par exemple dans EP-A-0326829, mais le procédé décrit fait appel à un solvant organique, par exemple l'éthanol, qui grâce à ses propriétés hydrophyle et lipophyle favorise la formation d'une phase unique. Ce procédé, qui utilise une lécithine de soja non fractionnée, présente cependant l'inconvénient de ne pas éviter l'apparition de problèmes de couleur, d'odeur et de floculation à l'entreposage.

Selon US-A-5084289, on forme des micelles en phase inversée, c'est à dire pour lesquelles la phase continue est la phase lipophyle, en dissolvant dans une huile un phospholipide, puis une petite quantité d'une solution aqueuse contenant un antioxydant hydrosoluble, par exemple la vitamine C, à haute concentration. On agite le mélange jusqu'à former des micelles inverses et on obtient ainsi une phase unique. La faible quantité de phase aqueuse relativement à la phase lipidique rend l'homogénéisation difficile, voire pratiquement impossible à grande échelle. De plus, il n'est possible d'incorporer par ce procédé que des substances fortement hydrosolubles, par exemple la vitamine C, mais pas des subtances peu hydrosolubles comme par exemple l'acide éthylènedinitrotétraacétique (EDTA). Enfin, ce procédé ne permet pas d'éviter l'apparition de couleurs et odeurs indésirables à l'entreposage du fait que la lécithine n'est pas fractionnée.

Nous avons trouvé de manière surprenante que ces inconvénients pouvaient être totalement éliminés lorsque l'on utilisait une fraction de lécithine pratiquement exempte de phosphatidylcholine.

L'invention concerne donc un procédé d'incorporation d'un principe actif hydrosoluble dans une matière grasse en présence de lécithine, caractérisé par le fait que l'on traite la lécithine de manière à produire une fraction de lécithine pratiquement exempte de phosphatidylcholine, que l'on incorpore au mélange de cette fraction et de la matière grasse sous forme liquide sous agitation vigoureuse une solution aqueuse du principe actif hydrosoluble en quantité suffisante pour assurer l'hydratation de la fraction de lécithine de manière à former un mélange hétérogène et que l'on sèche ce mélange qui devient alors homogène.

Dans le contexte de l'invention, on entend par "fraction de lécithine pratiquement exempte de phosphatidylcholine" une fraction obtenue à partir de lécithine commerciale, par exemple de soja, par un traitement permettant de séparer la phosphatidylcholine des autres constituants tels que la phosphatidyléthanolamine, le phosphatidylinositol et l'acide phosphatidique, ci-après dénommée "autres PL" (autres phospholipides).

Selon un premier mode de réalisation, on traite la lécithine commerciale en solution dans l'huile avec une terre décolorante à titre d'adsorbant, puis on sépare l'huile ainsi traitée.

Selon un second mode de réalisation, qui est préféré, on traite la lécithine en solution dans un mélange de solvants organiques par chromatographie liquide sur colonne de gel de silice de manière connue et on recueille la fraction contenant les autres PL, qui est également débarrassée de la majeure partie des triglycérides.

Les principes actifs hydrosolubles envisagés selon l'invention sont, par exemple, des agents cosmétiques ou dermatologiques ou antioxydants hydrosolubles. On peut citer, par exemple, les antioxydants d'usage courant, par exemple la vitamine C, les extraits végétaux, par exemple de romarin, de thé vert, les acides organiques, par exemple les hydroxyacides tel l'acide citrique, les acides phénoliques, par exemple les acides caféique, quiniques, chlorogéniques, la caféine, les acides aminés, les phénylindanes et les agents séquestrants, par exemple les citrates ou l'EDTA.

La matière grasse à protéger contre l'oxydation est riche en acides gras insaturés, particulièrement polyinsaturés. On peut citer les huiles végétales, par exemple de tournesol, de germes de blé, de pépins de raisins, de maïs, de carthame, d'olive, d'onagre, de bourrache et l'huile de pépins de cassis. Comme huile animale, on peut citer la graisse de poule, l'huile de beurre, les huiles d'animaux marins, en particulier de poisson.

L'incorporation du principe actif hydrosoluble a lieu sous agitation vigoureuse, à une température supérieure à 60°C et de préférence à environ 80°C. On peut l'introduire sous forme d'une solution aqueuse ou en variante sous forme sèche, puis ajouter de l'eau. La quantité d'eau dans le mélange doit être suffisante pour hydrater la quantité de fraction de lécithine qu'il contient. Elle représente 2 à 8 % en poids du mélange et, de préférence, environ 5 % de celui-ci. Cette opération a lieu pendant 10 à 30 min. et à l'abri de l'air, par exemple sous azote, ce qui résulte en la formation d'un mélange hétérogène.

On élimine ensuite l'eau du mélange hétérogène, par chauffage sous vide, de préférence à 60-90°C, avantageusement à 60-70°C et sous un vide de 0,5 à 35 mbar. Il en résulte la formation d'une phase micellaire homogène et stable.

Les exemples suivants illustrent l'invention. Dans ceux-ci, les parties et pourcentages sont en poids, sauf indication contraire.

### Exemple 1

On dissout dans une huile de tournesol (HTS) une lécithine de soja raffinée pauvre en métaux lourds (Topcithin 200 (R)) à 60 % de phospholipides (ci-après PL) sous azote.

On traite alors la solution avec une terre décolorante (Tonsil Optimum FF (R)) à 85-90°C sous une pression de 35 mbar pendant 30 min. en présence d'un agent antimousse (Rhodosil 70414 (R)) à raison d'une quantité d'adsorbant correspondant à 4 fois la quantité de phospholipides, puis on sépare la solution de l'adsorbant par filtration.

On ajoute ensuite à la solution les quantités indiquées au tableau 1 ci-après de vitamine C et de EDTA sous forme solide, puis 5 % d'eau déminéralisée. Après agitation vigoureuse à 80°C sous azote pendant 15 min., on obtient un mélange hétérogène que l'on sèche à la pression de 35 mbar, puis 0,5 mbar à 80-90°C pendant 30 min.et que l'on filtre. Il en résulte une phase micellaire homogène.

On a évalué l'huile stabilisée (1a) en comparaison avec la même huile non stabilisée (1b) et une huile stabilisée avec une lécithine commerciale n'ayant pas subi le traitement par la terre décolorante (1c):
- Du point de vue de leur stabilité à l'oxydation, mesurée avec le test d'oxydation accélérée Rancimat (R) à 110°C. Les temps d'induction obtenus, exprimés en h, représentent les valeurs OSI (Oil Stability Index).
- Du point de vue de leur stabilité à l'entreposage à 15°C par leur contrôle visuel et olfactif.

Les résultats sont consignés dans le tableau 1 ci-après:

**Tableau 1**

| Essai | PL mg/kg | Vit.C mg/kg | EDTA mg/kg | Traitement | OSI, 110°C, h | Couleur |
|---|---|---|---|---|---|---|
| 1a | 10000 | 1000 | 200 | oui | 19,5 | jaune |
| 1b | ---- | ---- | ---- | ---- | 5 | jaune clair |
| 1c | 10000 | 1000 | 200 | non | 17,5 | jaune orange |

On constate que le traitement de la lécithine avec une terre décolorante améliore la couleur et augmente la stabilité à l'oxydation.

### Exemples 2-10

On procède comme à l'exemple 1 à la stabilisation de HTS en utilisant conjointement la vitamine C et différents agents sequestrants en différentes quantités. A titre de comparaison, on mesure les valeurs OSI de l'huile non stabilisée (2a) et de l'huile contenant la fraction PL traitée avec l'adsorbant (2b).

A partir des valeurs OSI, on a calculé le facteur de protection (PF) qui correspond au quotient:
PF = OSI de l'huile stabilisée/OSI de l'huile non stabilisée, quotient qui représente l'augmentation du temps d'induction.

Les résultats sont indiqués dans le tableau 2 ci-après:

**Tableau 2**

| Exemple | PL mg/kg | Vit.C mg/kg | Agent séquestrant,A S | AS mg/kg | OSI 110°C, h | PF | Stabilité à 15°C après 5j |
|---|---|---|---|---|---|---|---|
| 2a | ---- | ---- | ---- | ---- | 5 | 1 | limpide |
| 2b | 5000 | ---- | ---- | ---- | 5,7 | 1,14 | limpide |
| 2 | 5000 | 1000 | ---- | ---- | 19 | 3,8 | légérement trouble |
| 3 | 5000 | 1000 | EDTA | 100 | 22,5 | 4,5 | légérement trouble |
| 4 | 5000 | 1000 | EDTA | 200 | 22 | 4,4 | limpide |
| 5 | 5000 | 1000 | EDTA | 400 | 20 | 4 | limpide |
| 6 | 5000 | 1000 | EDTA Na2 | 200 | 21 | 4,2 | légérement trouble |
| 7 | 5000 | 1000 | Acide citrique | 200 | 17 | 3,4 | légérement trouble |
| 8 | 5000 | 1000 | Acide citrique Na3 | 200 | 20,5 | 4,1 | légérement trouble |
| 9 | 5000 | 1000 | Acide citrique Na3 | 400 | 16 | 3,2 | limpide |
| 10 | 5000 | 1000 | EDTA | 200 | 21,8 | 4,36 | légérement trouble |
| | | | Acide citrique Na3 | 200 | | | |

On constate que l'utilisation de PL seule ne permet pas de stabiliser l'huile (2b comparé à 2a).

### Exemples 11-15

En procédant comme à l'exemple 1 précédent, on évalue la stabilité à l'entreposage, l'OSI et le PF de l'HTS stabilisée avec différents rapports Vit.C/PL. Les résultats sont indiqués dans le tableau 3 ci-après, en comparaison avec l'huile non protégée (11a) et avec la même huile à laquelle on a ajouté 1,66 % de mélange ternaire Vit.C-PL-Vit.E dans les proportions 1000 mg/kg Vit.C-4500 mg/kg PL-500 mg/kg vit.E (11b).

**Tableau 3**

| Exemple | PL mg/kg | Vit.C mg/kg | EDTA mg/kg | Vit.C/PL x100 | OSI 110°C, h | PF | Stabilité à 15°C après 5j |
|---|---|---|---|---|---|---|---|
| 11a | ---- | ---- | ---- | ---- | 5 | 1 | limpide |
| 11b | 4500 | 1000 +500 mg/kg Vit.E | ---- | 22 | 15,8 | 3,16 | légérement trouble |
| 11 | 2500 | 1000 | 200 | 40 | 14,7 | 2,94 | légérement trouble |
| 12 | 5000 | 1000 | 200 | 20 | 22 | 4,4 | légérement trouble |
| 13 | 6600 | 1000 | 200 | 15 | 20,8 | 4,16 | légérement trouble |
| 14 | 7500 | 1000 | 200 | 13,3 | 20 | 4 | limpide |
| 15 | 10000 | 1000 | 200 | 10 | 20 | 4 | limpide |

On constate que l'utilisation d'un mélange ternaire comprenant la Vit.E ne permet pas d'améliorer la stabilité de l'huile ni les propriétés antioxydantes par rapport à la mise en oeuvre d'un mélange PL-Vit.C (11b comparé à 11-15). On peut donc en conclure que la synergie entre les Vit.C et E intervient déjà avec la Vit.E contenue naturellement dans l'HTS (correspondant à 760 mg/kg). Donc, pour les huiles végétales contenant naturellement de la Vit.E, un apport supplémentaire de Vit.E n'est pas bénéfique.

On a procédé à l'entreposage pendant 1 mois à 15°C des huiles stabilisées et constaté que celles stabilisées avec un rapport Vit.C/PL supérieur à 13 % se sont troublées durant cette période. On voit donc qu'il est préférable que ce rapport soit < ou = à 13 % pour obtenir une stabilité optimale de l'huile.

### Exemples 16-19

En procédant comme à l'exemple 1 précédent, on évalue la stabilité à l'entreposage, l'OSI et le PF de l'HTS stabilisée avec différentes quantités de Vit.C, le rapport Vit.C/PL étant constant. Les résultats sont indiqués dans le tableau 4 ci-après, en comparaison avec l'huile non protégée (16a).

**Tableau 4**

| Exemple | PL mg/kg | Vit.C mg/kg | EDTA mg/kg | Vit.C/PL x100 | OSI 110°C, h | PF | Stabilité à 15°C après 1 mois |
|---|---|---|---|---|---|---|---|
| 16a | ---- | ---- | ---- | ---- | 5 | 1 | limpide |
| 16 | 2500 | 250 | 200 | 10 | 12,3 | 2,4 | limpide |
| 17 | 5000 | 500 | 200 | 10 | 16 | 3,2 | limpide |
| 18 | 7500 | 750 | 200 | 10 | 18 | 3,6 | limpide |
| 19 | 10000 | 1000 | 200 | 10 | 20 | 4 | limpide |

### Exemples 20-24

En procédant comme à l'exemple 1 à la stabilisation de l'huile de pépins de cassis (HPC), on évalue la stabilité à l'entreposage, l'OSI et le PF de l'HPC stabilisée avec un rapport constant Vit.C/PL. Les résultats sont indiqués dans le tableau 5 ci-après, en comparaison avec l'huile non protégée (20a) et avec la même huile à laquelle on a ajouté 1,66 % de mélange ternaire Vit.C-PL-Vit.E dans les proportions 1000 mg/kg Vit.C-4500 mg/kg PL-500 mg/kg vit.E (20b).

**Tableau 5**

| Exemple | PL mg/kg | Vit.C mg/kg | EDTA mg/kg | Vit.E mg/kg | Vit.C/PL x 100 | OSI 100°C, h | PF | Stabilité à 15°C après 20j. |
|---|---|---|---|---|---|---|---|---|
| 20a | ---- | ---- | ---- | ---- | ---- | 4 | 1 | limpide |
| 20b | 4500 | 1000 | ---- | 500 | 22 | 17,5 | 4,38 | légérement trouble |
| 20 | 10000 | 1000 | ---- | ---- | 10 | 19,8 | 4,95 | limpide |
| 21 | 10000 | 1000 | ---- | 500 | 10 | 17,6 | 4,4 | limpide |
| 22 | 10000 | 1000 | 400 | ---- | 10 | 24 | 6 | limpide |
| 23 | 10000 | 1000 | 400 | 500 | 10 | 20,2 | 5,05 | limpide |
| 24 | 5000 | 500 | 200 | ---- | 10 | 17,2 | 4,3 | limpide |

Les résultats confirment ceux obtenus avec l'HTS (exemples 11-15), à savoir que l'adjonction de Vit.E n'améliore pas la stabilité oxydative de l'HPC, mais aurait plutôt un effet prooxydant dans une huile végétale contenant naturellement environ 750 ppm de Vit.E (exemple 22 comparé à 23).

### Exemple 25

En procédant comme à l'exemple 1 à la stabilisation de la graisse de poule, exempte de Vit.E, on évalue l'OSI, le PF et la couleur avec et sans Vit.E ajoutée et en comparaison avec la graisse sans protection. Les résultats sont indiqués dans le tableau 6 ci-après.

**Tableau 6**

| PL mg/kg | Vit.C mg/kg | Vit.E mg/kg | EDTA mg/kg | OSI 120°C, h | PF | Couleur après Rancimat |
|---|---|---|---|---|---|---|
| ---- | ---- | ---- | ---- | 2 | 1 | jaune |
| 5000 | 500 | ---- | ---- | 7,3 | 3,65 | jaune |
| 5000 | 500 | 250 | ---- | 16,5 | 8,25 | orange |
| 5000 | 500 | ---- | 100 | 10,5 | 5,25 | jaune |
| 5000 | 500 | 250 | 100 | 19,3 | 9,65 | jaune |

Les résultats précédents confirment l'effet de synergie entre les Vit.C et E lorsque la matière grasse à protéger ne contient pas naturellement de Vit.E.

### Exemple 26

On dissout dans une HTS une lécithine de soja exempte de PC (fraction autres PL), qui a été obtenue par chromatographie liquide sur colonne de gel de silice en utilisant un mélange de solvants hexane/2-propanol/eau dans les proportions 1/1/0,1, sous forme de seconde fraction. On recueille également une première fraction riche en phosphatidylcholine (fraction PC).

On ajoute ensuite à l'huile les quantités 10000 mg/kg de chaque fraction de lécithine, 1000 mg/kg de Vit.C et 200 mg/kg d'EDTA sous forme solide, puis 5 % d'eau déminéralisée. Après agitation vigoureuse à 20°C sous azote pendant 15 min., on obtient un melange hétérogène que l'on sèche à la pression de 35 mbar, puis 0,5 mbar à 80-90°C pendant 30 min. Après filtration, il en résulte une phase micellaire homogène dans le cas de l'huile stabilisée avec la fraction autres PL. Par contre, la fraction PC n'a pas pu être dissoute dans l'huile.

On évalue ensuite l'OSI. Les résultats sont indiqués dans le tableau 7 ci-après.

**Tableau 7**

| Phospholipides | OSI 110°C | Remarques |
|---|---|---|
| Fraction PC | ---- | La fraction n'a pas pu être dissoute dans l'huile |
| Fraction autres PL | 21 | La fraction se dissout dans l'huile, qui est parfaitement limpide |

### Exemples 27-31

On a stabilisé une HTS avec différents principes actifs antioxydants hydrosolubles en suivant la procédure de l'exemple 26. Les résultats sont indiqués dans le tableau 8 ci-après. A titre comparatif, on a évalué l'HTS sans additif (27a).

Les phénylindanes ont été obtenus à partir du café en utilisant le procédé d'extraction faisant l'objet de la demande de brevet parallèle de la titulaire déposée ce jour sous le titre: "Phénylindanes, procédé de préparation et utilisations".

**Tableau 8**

| Exemple | PL mg/kg | Principe actif (PA) | PA mg/kg | EDTA mg/kg | OSI 110°C, h | PF |
|---|---|---|---|---|---|---|
| 27a | ---- | ---- | ---- | ---- | 5 | 1 |
| 27 | 10000 | L-Histidine | 1000 | 200 | 11,5 | 2,3 |
| 28 | 10000 | L-Cystéine | 1000 | 200 | 12 | 2,4 |
| 29 | 10000 | Extrait de romarin | 1000 | 200 | 14 | 2,8 |
| 30 | 10000 | Extrait de thé vert | 1000 | 200 | 12 | 2,4 |
| 31 | 10000 | Phénylindanes | 500 | ---- | 30,5 | 6,1 |

### Exemples 32-33

Pour stabiliser une HTS, on met en oeuvre le procédé décrit à l'exemple 1 précédent jusqu'à l'hydratation des PL. Au stade du séchage, on réalise l'opération à différentes températures. On évalue les propriétés antioxydantes ainsi que la couleur des huiles stabilisées en comparaison avec l'huile non stabilisée (32a). Les résultats sont indiqués dans le tableau 9 ci-après.

**Tableau 9**

| Exemple | Température de séchage | PL sans PC | Vit.C mg/kg | EDTA mg/kg | OSI 110°C, h | PF | Mesure de la couleur Lovibond 5,25'' | |
|---|---|---|---|---|---|---|---|---|
| | | | | | | | Y | R |
| 32a | ---- | ---- | ---- | ---- | 5 | 1 | 8,4 | 1,3 |
| 32 | 80-90°C | 10000 | 1000 | 400 | 21 | 4,2 | 43 | 4,8 |
| 33 | 60-70°C | 10000 | 1000 | 400 | 22,8 | 4,56 | 18,5 | 2,7 |

On constate que la couleur est moins intense dans le cas d'un séchage à température plus basse.

## Revendications

1. Procédé d'incorporation d'un principe actif hydrosoluble dans une matière grasse en présence de lécithine, **caractérisé par le fait que** l'on traite la lécithine de manière à produire une fraction de lécithine pratiquement exempte de phosphatidylcholine, que l'on incorpore au mélange de cette fraction et de la matière grasse sous forme liquide sous agitation vigoureuse une solution aqueuse du principe actif hydrosoluble en quantité suffisante pour assurer l'hydratation de la fraction de lécithine de manière à former un mélange hétérogène et que l'on sèche ce mélange qui devient alors homogène.

2. Procédé selon la revendication 1, **caractérisé par le fait que** l'on traite la lécithine en solution dans l'huile avec une terre décolorante à titre d'adsorbant, puis on sépare l'huile ainsi traitée.

3. Procédé selon la revendication 1, **caractérisé par le fait que** l'on traite la lécithine en solution dans un mélange solvant par chromatographie liquide sur colonne de manière connue et on recueille la fraction contenant les phospholipides autres que la phosphatidylcholine, qui est également débarrassée de la majeure partie des triglycérides.

4. Procédé selon la revendication 1, **caractérisé par le fait que** les principes actifs hydrosolubles envisagés sont des agents cosmétiques ou dermatologiques ou antioxydants hydrosolubles, notamment les antioxydants d'usage courant comme la vitamine C, les extraits végétaux, les acides organiques, les acides aminés et les agents séquestrants.

5. Procédé selon la revendication 1, **caractérisé par le fait que** la matière grasse à protéger contre l'oxydation est riche en acides gras insaturés, notamment une huile de tournesol, de germes de blé, de pépins de raisins, de maïs, de carthame, d'olive, d'onagre, de bourrache, de pépins de cassis, la graisse de poule, l'huile de beurre ou une huile d'animaux marins.

6. Procédé selon la revendication 1, **caractérisé par le fait que** l'incorporation du principe actif hydrosoluble a lieu sous agitation vigoureuse, à une température supérieure à 60°C.

7. Procédé selon la revendication 1, **caractérisé par le fait que** l'on incorpore le principe actif sous forme d'une solution aqueuse ou sous forme sèche, puis on ajoute de l'eau dans le mélange, en quantité suffisante pour hydrater la quantité de fraction de lécithine qu'il contient.

8. Procédé selon la revendication 7, **caractérisé par le fait que** l'eau représente de 2 à 8 % en poids du mélange et que l'hydratation a lieu pendant 10 à 30 min. et à l'abri de l'air, ce qui résulte en la formation d'un mélange hétérogène.

9. Procédé selon la revendication 7, **caractérisé par le fait que** l'on élimine l'eau du mélange hétérogène, par chauffage à 60-90°C et sous un vide de 0,5 à 35 mbar, pour obtenir une phase micellaire homogène.

## Patentansprüche

1. Verfahren zur Einarbeitung eines wasserlöslichen Wirkstoffs in ein Fett in Gegenwart von Lecithin, **dadurch gekennzeichnet, dass** man Lecithin so behandelt, dass eine praktisch phosphatidylcholinfreie Lecithinfraktion hergestellt wird, dass man in die Mischung aus dieser Fraktion und Fett in flüssiger Form unter kräftigem Rühren eine wässrige Lösung des wasserlöslichen Wirkstoffs in ausreichender Menge einarbeitet, um die Hydratisierung der Lecithinfraktion zu gewährleisten, so dass eine heterogene Mischung gebildet wird, und dass man diese Mischung trocknet, wobei diese homogen wird.

2. Verfahren nach Anspruch 1, **dadurch gekennzeichnet, dass** man Lecithin in Lösung in Öl mit Bleicherde als Adsorbtionsmittel behandelt und dann das auf diese Weise behandelte Öl abtrennt.

3. Verfahren nach Anspruch 1, **dadurch gekennzeichnet, dass** man Lecithin in Lösung in einer Lösemittelmischung auf bekannte Weise durch Flüssigkeits-Säulenchromatographie behandelt und die die anderen Phospholipide als Phosphatidylcholin enthaltende Fraktion gewinnt, die ebenfalls von dem größten Teil der Triglyceride befreit wird.

4. Verfahren nach Anspruch 1, **dadurch gekennzeichnet, dass** die in Betracht gezogenen wasserlöslichen Wirkstoffe kosmetische oder dermatologische Mittel oder wasserlösliche Antioxidantien, insbesondere üblicherweise verwendete Antioxidantien wie Vitamin C, Pflanzenextrakte, organische Säuren, Aminosäuren und Sequestriermittel sind.

5. Verfahren nach Anspruch 1, **dadurch gekennzeichnet, dass** das gegen Oxidation zu schützende Fett reich an ungesättigten Fettsäuren ist und insbesondere Sonnenblumenöl, Weizenkeimöl, Traubenkernöl, Maisöl, Safloröl, Olivenöl, Nachtkerzenöl, Borretschöl, Kernöl von schwarzen Johannisbeeren, Hühnerfett, Butteröl oder Öl von Meerestieren ist.

6. Verfahren nach Anspruch '1, **dadurch gekennzeichnet, dass** die Einarbeitung des wasserlöslichen Wirkstoffs unter kräftigem Rühren bei einer Temperatur über 60°C stattfindet.

7. Verfahren nach Anspruch 1, **dadurch gekennzeichnet, dass** man den Wirkstoff in Form einer wässrigen Lösung oder in trockener Form einarbeitet und dann der Mischung Wasser in ausreichender Menge beigibt, um die in ihr enthaltene Menge an Lecithinfraktion zu hydratisieren.

8. Verfahren nach Anspruch 7, **dadurch gekennzeichnet, dass** das Wasser 2 bis 8 Gew.-% der Mischung darstellt und dass die Hydratisierung 10 bis 30 min unter Luftabschluss stattfindet, was zur Bildung einer heterogenen Mischung führt.

9. Verfahren nach Anspruch 7, **dadurch gekennzeichnet, dass** man das Wasser aus der heterogenen Mischung durch Erhitzung auf 60-90°C und unter einem Unterdruck von 0,5 bis 35 mbar entfernt, um eine homogene mizelläre Phase zu erhalten.

## Claims

1. Method for incorporating a water-soluble active principle into a fat in the presence of lecithin, **characterized in that** lecithin is treated in such a way as to produce a lecithin fraction practically free from phosphatidylcholine, that an aqueous solution of the water-soluble active principle in a sufficient quantity to ensure hydration of the lecithin fraction is incorporated with vigorous stirring into the mixture of this fraction and the fat in liquid form so as to form a heterogeneous mixture, and that this mixture is dried and then becomes homogeneous.

2. Method according to claim 1, **characterized in that** lecithin is treated in solution in oil with a decolorizing earth used as an adsorbent, and the oil treated in this way is then separated.

3. Method according to claim 1, **characterized in that** lecithin is treated in solution in a solvent mixture by liquid chromatography on a column in a known manner and the fraction is collected containing phospholipids other than phosphatidylcholine, from which the major part of the triglycerides is removed.

4. Method according to claim 1, **characterized in that** the water-soluble active principles considered are water-soluble cosmetic, dermatological or antioxidant agents, in particular antioxidants currently used, such as vitamin C, vegetable extracts, organic acids, amino acids and sequestering agents.

5. Method according to claim 1, **characterized in that** the fat to be protected against oxidation is rich in unsaturated fatty acids, in particular sunflower oil, wheat germ oil, grape seed oil, corn oil, safflower oil, olive oil, evening primrose oil, borage oil, blackcurrant seed oil, chicken fat, butter oil or oil from marine animals.

6. Method according to claim 1, **characterized in that** the water-soluble active principle is incorporated with vigorous stirring at a temperature above 60°C.

7. Method according to claim 1, **characterized in that** the active principle is incorporated in the form of an aqueous solution or in a dry form, and water is then added to the mixture in a sufficient quantity to hydrate the quantity of lecithin fraction that it contains.

8. Method according to claim 7, **characterized in that** water represents 2 to 8 % by weight of the mixture and that hydration takes place during 10 to 30 min with the exclusion of air, this resulting in the formation of a heterogeneous mixture.

9. Method according to claim 7, **characterized in that** water is removed from the heterogeneous mixture by heating at 60-90°C under a vacuum of 0.5 to 35 mbar, so as to obtain a homogeneous micellar phase.
